Europäisches Patentamt
European Patent Office
Office européen des brevets

(19)

(11) Publication number: **0 149 592**
**A2**

# (12) EUROPEAN PATENT APPLICATION

(21) Application number: 85850019.2

(22) Date of filing: 17.01.85

(51) Int. Cl.[4]: C 07 D 241/24
A 61 K 31/495

(30) Priority: 19.01.84 FI 840209

(43) Date of publication of application: 24.07.85 Bulletin 85/30

(84) Designated Contracting States: AT BE CH DE FR GB IT LI LU NL SE

(71) Applicant: LÄKEMEDELSFABRIKEN-MEDICA AB
Industrigatan 23-25
SF-00510 Helsingfors 51(FI)

(72) Inventor: Dahlström, Mikael
Messeniusgatan 7 A 20
SF-00250 Helsingfors 25(FI)

(72) Inventor: Malmen, Yngve
Timmerdammsvägen 5 B 14
SF-00640 Helsingfors 64(FI)

(74) Representative: Onn, Thorsten et al,
AB STOCKHOLMS PATENTBYRA Box 3129
S-103 62 Stockholm(SE)

## (54) Process for the preparation of a pharmacologically active sulphonylurea derivative.

(57) The present invention relates to a method of preparing N-{4-[2-(5-methylpyrazinyl-2-carboxamido)-ethyl]-benzenesulphonyl}-N'-cyclohexylurea or glipizide, which has the chemical structure (I),

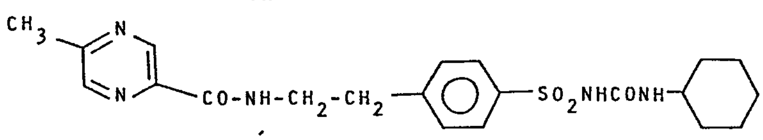

I

by letting 4-[2-(5-methylpyrazinyl)-2-carboxamido)-ethyl]-benzenesulphonamide with the chemical structure (II), where $R^1 = NH_2$, react with N-cyclohexyl trichloroacetamide with the chemical structure (III), where $R^2 = NHCOCCl_3$, according to the following reaction scheme:

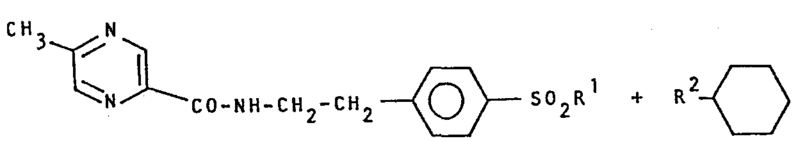

II III

./...

EP 0 149 592 A2

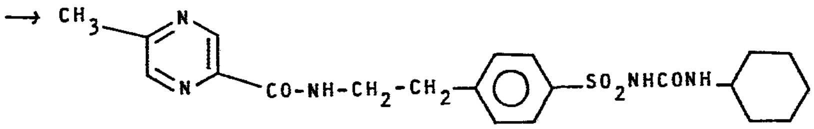

I

The reaction is carried out in the presence of a polar, aprotic solvent such as N-methylpyrrolidone (NMP), N,N-dimethylformamide (DMF), N,N-dimethylacetamide (DMA) or dimethylsulphoxide (DMSO) under anhydrous conditions in the presence of a base such as an alcoholate, amide or hydride of an alkali metal. The reaction mixture is heated under reflux condensation to 40-100°C and the reaction is going on for 2-5 hours.

PROCESS FOR THE PREPARATION OF A PHARMACOLOGICALLY ACTIVE SULPHONYLUREA DERIVATIVE

The present invention relates to a process for the preparation of N-{4-[2-(5-methylpyrazinyl-2-carboxamido)-ethyl]-benzenesulphonyl}-N'-cyclohexylurea or glipizide, which has the chemical structure:

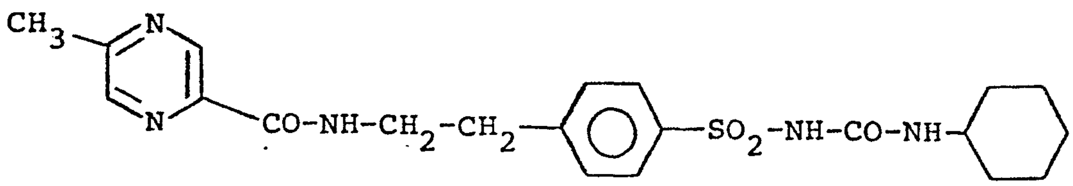

I

This compound is used in medicine as a potent blood glucose reducing agent for the treatment of diabetes.

Like some other newer, antidiabetically active sulphonylurea derivatives, e.g. glibenclamide, glipizide belongs to the so-called second-generation derivatives, characterised by being therapeutically active in doses as low as 2.5-10 mg/day, while the classic sulphonylurea derivatives are used in doses of 100-150 mg or even several grams daily and the biguanide derivatives are used in corresponding doses.

Chemically, glipizide is characterised by that the molecule contains a 2-methylpyrazinyl group, which makes it extremely difficult to synthetise at a good yield, in contrast to many other sulphonylurea derivatives in the market.

The compound prepared according to the method described in the present application has been previously prepared by different methods.

The preparation of glipizide according to the Finnish patent No. 52720, can be illustrated by the following general reaction scheme:

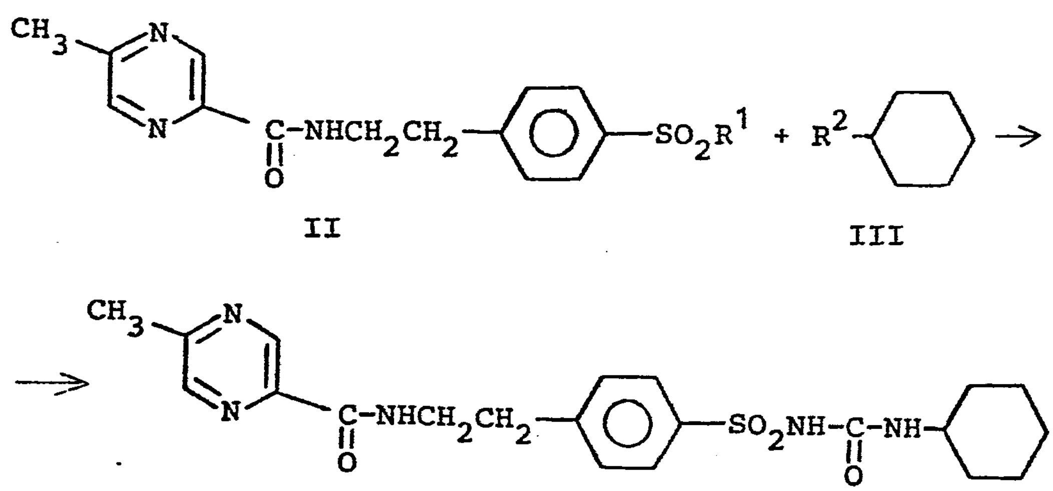

II

III

I

where $R^1$ = $NH_2$, -NCO, $-NHCONH_2$ or $-NHCOOC_2H_5$

and $R^2$ = $-NH_2$ or -NCO-

The starting substance II, where $R^1$ = $-NH_2$, is difficult to synthetise at a good yield and is considerably more expensive than the starting substance III.

Thus, the synthesis is significantly less profitable when II (where $R^1$ = $-NH_2$, i.e. the sulphonamide) is used as starting substance for the preparation of intermediate products of a type where $R^1$ may be either -NCO, $-NCONH_2$ or $-NHCOOC_2H_5$. In these synthese the compound of type II (where $R^1$ = $-NH_2$, i.e. the sulphonamide) is involved in one additional step. The compounds thus produced are utterly unstable difficult to handle.

Compound III, where $R^2$ = $-NH_2$, i.e. cyclohexylamine is generally available in the market and is used as raw material for the preparation of compounds of type III, where $R^2$ = -NCO. This is done by reacting cyclohexylamine with phosgene, which like cyclohexyl isocyanate is a very unpleasant substance, difficult to handle with regard to safety at work.

The other process described in the same patent follows the reaction formula:

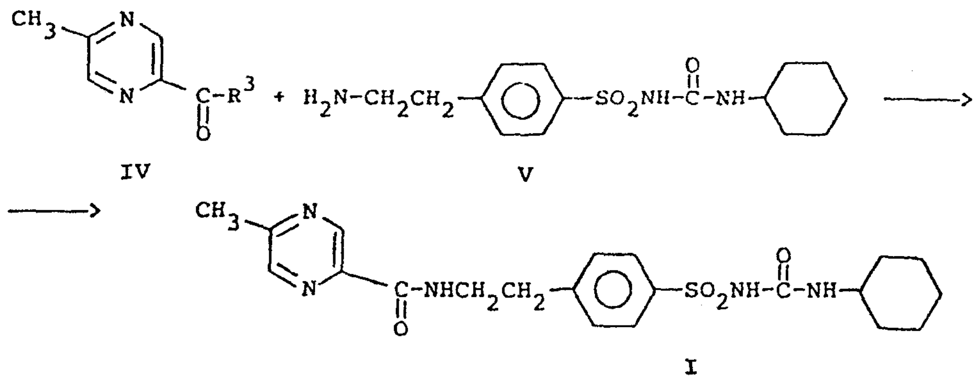

IV V I

where $R^3$ = -OH, -Cl, $-OC_2H_5$, $-NH_2$

and -OCOR, which last-mentioned together with the carbonyl group forms an undefined mixture of anhydrides.

In this process compounds of type IV are allowed to react with compounds of type V. The method has the disadvantage of both reactants being very expensive, at the same time as the yield of the desired final product is low.

According to the above-mentioned application the most practical way of preparing N-{4-[2-(5-methylpyrazinyl-2-carboxamido)-ethyl]-benzenesulphonyl}-N'-cyclohexylurea or glipizide is to suspend-4-[2-(5-methylpyrazine-2-carboxamido)-ethyl]-benzenesulphonamide (II) in a mixture of 2 M NaOH and acetone, add to the suspension cyclohexyl isocyanate (III) dropwise at a temperature of 0,5°C and allow the reaction mixture to stand at room temperature for 3 hours, after which water is added, insoluble matter is filtered off and the product is precipitated by acidification with hydrochloric acid. After filtration and recrystallisation a product is obtained which melts at 200-203°C.

This method is unsatisfactory and gives a poor yield, because the water present in the reaction mixture causes hydrolysis of both the reactants. Cyclohexyl isocyanate is the more sensitive of the two. It is easily hydrolysed into cyklohexylamine which, again reacts with a molecule of cyclohexyl isocyanate so that N,N'dicyclohexylurea is formed in considerable amounts. As was mentioned in the foregoing, cyclohexyl isocyanate is, moreover, an extremely unpleasant reagent, which causes irritaion of the skin and the mucous membranes comparable to the effects of lacrimatory gases.

The German patent application Offenlegungsschrift DE 2 213 602 describes a method of preparing N-{4-/2--(5-methylpyrazinyl-2-carboxamido)-ethyl7-benzenesulphonyl}-N'-cyclohexylurea or glipizide by letting a compound with a structure of type II, where $R^1$ = $-NH_2$, i.e., the sulphonamide react with a compound of type III, where $R^2$ = $-NHCOOCl_3$. The reaction is carried out under stirring at 140-160°C, without a solvent, in the presence of $K_2CO_3$.

The disadvantage of this method is the high reaction temperature at which the unstable reactants largely decompose. Consequently, the yield of the desired final product is relatively low, only about 20 % of the theoretical yield, and the product has a high content of impurities in the form of decomposition products and unreacted starting material, which makes it difficult to purify and further reduces the yield and the profitability.

In the above-mentioned German application, it is also mentioned that hydroxides can be used as bases in the reaction instead of $K_2CO_3$. However, as hydroxides cause the presence of water in the reaction mixture, the hydrolysis of the reactants greatly increases at the prevailing high reaction temperature and the reaction mixture will thus have a high content of undesired decomposition products.

Solvents such as alcohols, ethers and ketones have also been used in the reaction described above. In experiments with these solvents we have found only traces of the desired product in the reaction mixture. The reason for this is that compounds with a structure of type II,

where $R^1$ = NHX and X = an alkali metal or earth alkali metal, are practically insoluble in the above mentioned solvents. Thus, the contact between the reactants is unsatisfactory.

If the synthesis is carried out as described above at a reaction temperature of 150°C, the resulting product is not a melt but a crystalline mass which is difficult to process. As the mass gradually hardens into a cake, the chance of getting o good yield of the desired product on a technical scale is even further reduced.

The method for preparation of glipizide according to the present application differs considerably from those described above.

We have unexpectedly found that, by careful selection of the solvent and the base, the drawbacks mentioned above can be completely eliminated in the preparation of glipizide. The method according to the present invention gives an almost quantitative yield of chemically very pure glipizide.

The method invented by us is characterized by that N-{4-[2-(5-methylpyrazinyl-2-carboxamido(-ethyl]-benzenesulphonyl}-N'-cyclohexylurea with the structural formula I:

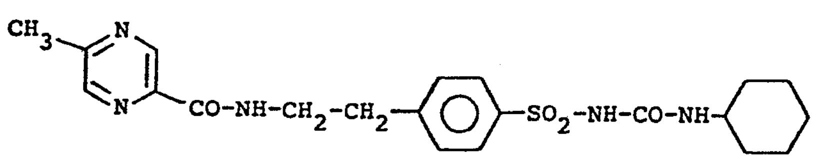

I

is prepared by letting 4-[2-(5-methylpyrazinyl-2-carboxamido)-ethyl]-benzenesulphonamide with the structural formula II, where $R^1$ = $NH_2$, react with N-cyclohexyl trichloroacetamide with the structural formula III, where $R^2$ = $NHCOCCl_3$, according to the following reaction scheme:

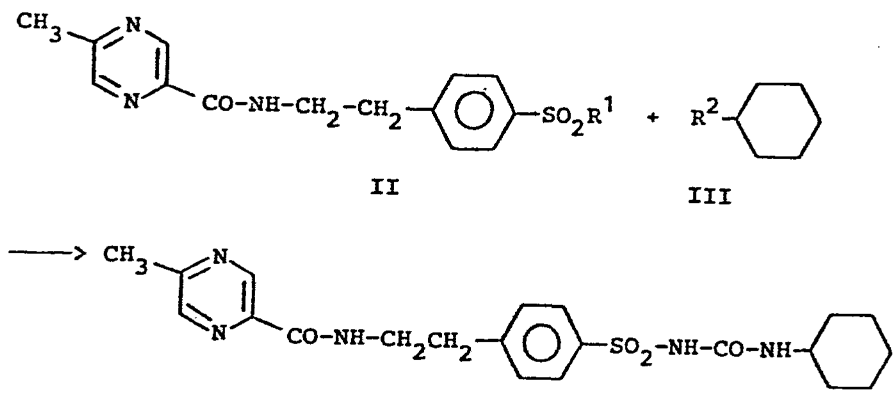

II III

I

in the presence of a polar, aprotic solvent such a N-methylpyrrolidone (NMP), N,N-dimethylformamide (DMF), N'N-dimethylacetamide (DMA) or dimethylsulphoxide (DMSO). The reaction must be carried out under strictly anhydrous conditions in the presence of a base such as an alcoholate, amide or hydride of an alkali metal.

The reaction can be carried out at a low temperature 40-100°C, preferably 50-80°C, and the reaction time is relatively short, 2-5 hours depending on the solvent used.

If, for instance, a lower alcohol, tetrahydrofurane or pyridine is used as solvent, the reaction mixture will contain only traces of the desired final product.

The method according to the present invention has the advantage of starting from the cheapest possible materials and not requiring the use of poisonous isocyanates or other substances difficult to handle, particularly on a technical scale. The reaction can be carrid out at a low temperature, which minimizes the risk of thermal decomposition. Thanks to the strictly product is avoided and they remain in solution throughout the reaction, which will thus be more complete. The final product obtained under the reaction conditions described above is very easily purified because of the low content of decomposition products and other by-products. The yield of glipizide is almost quantitative and much greater than the yield obtained when using the previously described methods.

The invention is best illustrated by the following

examples:

Example 1.

To 6.4 g of 4-/2-(5-methylpyrazinyl-2-carboxamido)--ethyl7-benzenesulphonamide and 5.5 g of N-cyclohexyl trichloroacetamide in 40 ml of dimethylsulphoxide (DMSO) there is added 5 ml of 30 % sodium methylate in methanol by portions, over a period of 2 hours and at a temperature of 50°C. The mixture is stirred for 3 more hours att 50°C, cooled, and 150 ml of cold water is added. Possible undissolved matter is filtered off and the filtrate is acidified with acetic acid to pH = 4.0, at which glippzide precipitates. The yield of final product after filtration and drying is 8.4 g (94.4 % of the theoretical yield). If necessary, the product is purified by recrystallisation to a melting point of 208-210°C.

Exempel 2.

To a solution of 6.4 g of 4-/2-(5-methylpyrazinyl-2-carboxamido)-ethyl7-benzenesulphonamide and 6.0 g of N-cyclohexyl trichloroacetamide in 50 ml of N,N'-dimethylacetamide (DMA) there is added 1.0 g of sodium amide at 60°C. The reaction is allowed to continue for 4 hours at the same temperature, after which the mixture is cooled and 100 ml of cold water is added. Any undissolved matter in the reaction mixture is filtered off and the filtrate is acidified with acetic acid to pH 4.0 at which glipizide precipitates. Yield after filtration and drying 7.6 g (85 % of theoretical). If necessary, the product is purified by recrystallisation to a melting point of 208-210°C.

Exemple 3

To a solution of 6.4 g of 4-/2-(5-methylpyrazinyl-2-carboxamido)-ethyl7-benzenesulphonamide and 5.5 g of N-cyclohexyl trichloroacetamide in 40 ml of N-methylpyrrolidone (NMP) there is added 5 ml of a 30 % solution of sodium methylate in methanol over a period of 1-2 hours at 70°C. The reaction is allowed to continue for one more

hour at the same temperature, after which 80 ml of cold water is added. Any undissolved matter is filtered off and the filtrate is acidified with acetic acid to pH 4.0, at which glipizide precipitates. Yield after filtration and drying 8.0 (90 % of theoretical). If necessary, the product is purified by recrystallisation to a melting point of 208-210°C.

Example 4

To a solution of 6.4 g of 4-/2-(5-methylpyrazinyl-2-carboxamido)-ethyl/-benzenesulphonamide and 5.5 g of N-cyclohexyl trichloroacetamide in 80 ml of N,N-dimethylformamide (DMF) there is added 1.1 g of NaH (55-60 % in oil) ny portions over a period of 30 minutes at 90°C. The reaction is allowed to continue for one more hour at the same temperature, after which 160 ml of cold water is added. Possible undissolved matter is filtered off and the filtrate is acidified with acetic acid to pH 4.0, at which glipizide precipitates. Yield after filtration and drying 7.6 g (85 % of theoretical). If necessary, the product is purified by recrystallisation to a melting point of 208-210°C.

-----------

PATENT CLAIMS

Process for the preparation of N-{4-[2-(5-methylpyrazinyl-2-carboxamido)-ethyl]-benzenesulphonyl}-N'-cyclohexylurea, a blood glucose reducing compound with the chemical structure (I),

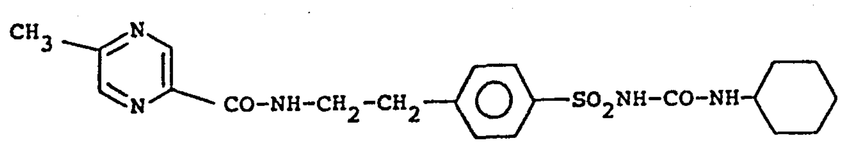

I

in which 4-[2-(5-methylpyrazinyl-2-carboxamido)-ethyl]-benzenesulphonamide with the chemical structure (II)

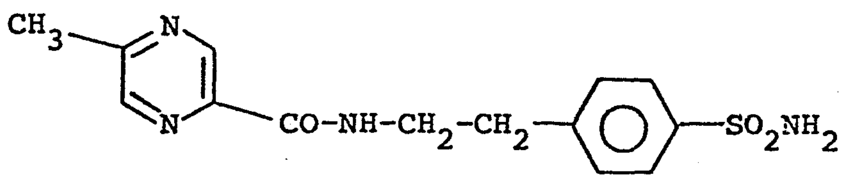

II

is allowed to react with N-cyclohexyl trichloroacetamide with the chemical structure (III),

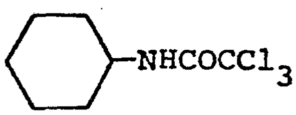

III

c h a r a c t e r i z e d by that the reaction is carried out in the presence of a polar, aprotic solvent such as N-methylpyrrolidone (NMP), N,N-dimethylformamide (DMF), N,N-dimethylacetamide (DMA) or dimethylsulpoxide (DMSO) under anhydrous conditions and in the presence of a base such as an alcoholate, amide or hydride of an

alkali metal, under reflux at a reaction temperature of 40-100°C, preferably 50-80°C, during 2-5 hours.

-----